# DEMANDE DE BREVET EUROPEEN

(11) **EP 2 017 199 A1**
(43) Date de publication de la demande: **21.01.2009**
(21) Numéro de dépôt: 08305120.1
(22) Date de dépôt: 23.04.2008
(51) Int. Cl.: B65D 85/42

(54) **Dispositif de conditionnement de produits biologiques**

(30) Priorité: 20.07.2007 FR 0756648
(71) Demandeur: L'AIR LIQUIDE, Société Anonyme pour l'Etude et l'Exploitation des Procédés Georges Claude, 75007 Paris (FR)
(72) Inventeur: Malboze, Frédéric, 77860 Quincy Voisins (FR); Hernandez Philippe, 94300 Vincennes (FR); Mitchell, Edward, 75014 Paris (FR); Sermet, Eric, 310001 Hangzhou (CN)
(74) Mandataire: De Cuenca, Emmanuel Jaime

(57) **Abrégé**

Dispositif de conditionnement (2) de produits biologiques comprenant un emballage (3) parallélépipédique ayant une face supérieure (103), une face inférieure (113), et deux faces latérales (123, 133) s'étendant selon une direction longitudinale (L) entre deux faces d'extrémité (143, 153) et définissant au moins un premier logement (9), le dispositif comportant un élément de stockage (4) distinct destiné à être logé de façon amovible dans le premier logement (9) de l'emballage (3) selon une direction parallèle à la direction longitudinale (L), l'élément de stockage (4) comprenant une zone de stockage d'un premier type de contenant de produits biologiques, l'élément de stockage (4) et/ou l'emballage (3) étant conformés pour délimiter au moins un second logement (10) pour un second type de contenant de produits biologiques, l'emballage (3) comprenant une ouverture sur une face d'extrémité (153) prévue pour l'introduction ou la sortie de l'élément de stockage (4) et/ou du second type de contenant, le dispositif comprenant un couvercle distinct de l'emballage (3) pour obturer sélectivement ladite ouverture.

## Description

La présente invention concerne un dispositif de conditionnement de produits biologiques.

Dans le cadre de prélèvements de produits biologiques d'un patient ou d'un donneur, les produits biologiques prélevés sont souvent stockés dans différents contenants, tels que des poches, des tubes ou des paillettes de prélèvement.

Des données relatives au patient ou au donneur et au membre du personnel médical ayant effectué le prélèvement doivent être apposées sur chaque contenant par ce dernier ou par un autre membre du personnel médical.

Cette apposition de données sur les différents contenants est fastidieuse pour le personnel médical et augmente de manière importante le temps opératoire. De plus, compte tenu du nombre important de contenants, les risques d'erreur dans les données apposées sur ces derniers ne sont pas négligeables.

Il doit être noté que ces différents contenants sont stockés à différents emplacements, ce qui complique leur localisation lorsque des essais médicaux doivent être effectués sur les produits biologiques contenus dans ceux-ci et engendre un volume de stockage important.

La présente invention vise à remédier à tout ou partie de ces inconvénients, et de manière avantageuse consiste à fournir un dispositif de conditionnement de produits biologiques qui soit de structure simple et économique, tout en facilitant le conditionnement de ces produits biologiques et en diminuant le volume de stockage de ces derniers.

A cet effet, l'invention concerne un dispositif de conditionnement de produits biologiques comprenant un emballage parallélépipédique ayant une face supérieure, une face inférieure, et deux faces latérales s'étendant selon une direction longitudinale entre deux faces d'extrémité et définissant au moins un premier logement, le dispositif comportant un élément de stockage distinct destiné à être logé de façon amovible dans le premier logement de l'emballage selon une direction parallèle à la direction longitudinale, l'élément de stockage comprenant une zone de stockage d'un premier type de contenant de produits biologiques ; l'élément de stockage et/ou l'emballage étant conformés pour délimiter au moins un second logement pour un second type de contenant de produits biologiques, l'emballage comprenant une ouverture sur une face d'extrémité prévue pour l'introduction ou la sortie de l'élément de stockage et/ou du second type de contenant, le dispositif comprenant un couvercle distinct de l'emballage pour obturer sélectivement ladite ouverture.

Ainsi, le dispositif de conditionnement selon l'invention permet le conditionnement d'au moins deux types de contenants différents, tels que par exemple le conditionnement de tubes et de poches de prélèvement.

Cette disposition permet d'identifier un nombre important de contenants en apposant des données uniquement sur la surface extérieure de l'emballage du dispositif de conditionnement.

Il en résulte une diminution du temps opératoire et des risques d'erreur d'identification des différents contenants.

En outre, cette disposition permet de rassembler à un même emplacement les différents contenants relatifs à un même patient, ce qui facilite la gestion de l'ensemble des contenants.

De préférence, les deux zones de stockage des premier et second types de contenant sont séparées l'une de l'autre par des moyens de séparation.

Selon un premier mode de réalisation de l'invention, l'emballage comprend au moins un élément de séparation conçus pour diviser l'emballage en au moins un premier et un second logements, le premier logement étant destiné à recevoir l'élément de stockage et le second logement formant la zone de stockage du second type de contenant de produits biologiques.

Avantageusement, le ou les éléments de séparation comportent une paroi de séparation s'étendant sur sensiblement toute la longueur de l'emballage selon la direction longitudinale.

Selon un second mode de réalisation de l'invention, l'élément de stockage comprend une première zone de stockage du premier type de contenant de produits biologiques et une seconde zone de stockage du second type de contenant de produits biologiques.

Avantageusement, la seconde zone de stockage est délimitée par un logement ménagé dans l'élément de stockage (lui-même).

De préférence, l'élément de stockage est réalisé en une pièce et obtenu par pliage d'une feuille comprenant une portion centrale, une première portion latérale reliée à la portion centrale par une première portion intermédiaire, et une seconde portion latérale reliée à la portion centrale par une seconde portion intermédiaire, la seconde zone de stockage étant délimitée, après pliage de la feuille, par la portion centrale, la première portion latérale et les deux portions intermédiaires.

Selon une autre caractéristique de l'invention, la première zone de stockage est délimitée, après pliage de la feuille, par les première et seconde portions latérales, la seconde portion latérale comportant au moins une rangée d'ouvertures destinées à former des moyens de fixation du premier type de contenant.

Selon une caractéristique de l'invention, la zone de stockage du second type de contenant de produits biologiques est conçue pour recevoir une poche de prélèvement de produits biologiques.

Selon une autre caractéristique de l'invention, l'élément de stockage comprend des organes de fixation du premier type de contenant de produits biologiques, ces organes de fixation étant agencés pour recevoir au moins un contenant tubulaire, tel qu'un tube de prélèvement de produits biologiques.

Avantageusement, l'emballage est une boîte sensiblement parallélépipédique comprenant des moyens d'ouverture.

Selon d'autres particularités possibles :
- le couvercle comprend un capuchon ayant la forme d'un tube obturé à une de ses extrémités et conformé pour recouvrir sélectivement l'ouverture et une portion de l'emballage selon la direction longitudinale,
- l'emballage a la forme générale d'un tube clos à une de ses extrémités, les faces d'extrémité de l'emballage ayant chacune une surface inférieure ou égale à la surface de chacune des autres faces de l'emballage,
- la bordure de la face ouverte du capuchon est biseautée par rapport à l'axe perpendiculaire à la direction de mise en place ou de retrait sur l'emballage,
- une partie de la paroi de séparation fait saillie par rapport à l'une des faces de l'emballage selon la direction longitudinale au niveau de l'ouverture,
- le capuchon a une section transversale conjuguée de la section transversale de l'emballage,
- l'emballage et/ou le capuchon comporte des orifices répartis selon la direction longitudinale, de préférence à proximité des faces latérales pour permettre la circulation de fluide cryogénique dans le dispositif.

De toute façon l'invention sera bien comprise à l'aide de la description qui suit en référence au dessin schématique annexé représentant, à titre d'exemples non limitatifs, deux formes d'exécution de ce dispositif de conditionnement.

La figure 1 est une vue en perspective d'un dispositif de conditionnement selon une première forme d'exécution.

La figure 2 est un vue en perspective du dispositif de conditionnement de figure 1 dans lequel sont stockés des tubes de prélèvement et une poche de prélèvement.

La figure 3 est une vue en perspective d'un dispositif de conditionnement selon une seconde forme d'exécution.

La figure 4 est une vue de dessus, à l'état déplié, de l'élément de stockage du dispositif de conditionnement de la figure 3.

La figure 5 est une vue du dispositif de conditionnement de figure 2 dans lequel sont stockés des tubes de prélèvement et une poche de prélèvement.

La figure 6 est une vue de dessus d'un troisième mode de réalisation possible du dispositif de conditionnement selon l'invention avec le couvercle en position ouverture par rapport à l'emballage.

La figure 7 est une vue analogue à la figure 6 avec le couverlce en position fermée par rapport à l'emballage.

La figure 8 est une vue en perspective, de la face arrière de l'emballage de la figure 7 en position ouverte.

La figure 9 est une vue de face de la face avant ouverte de l'emballage de la figure 7.

La figure 10 est une vue en perspective de la face arrière du couvercle de la figure 6 ou 7 en position ouverte.

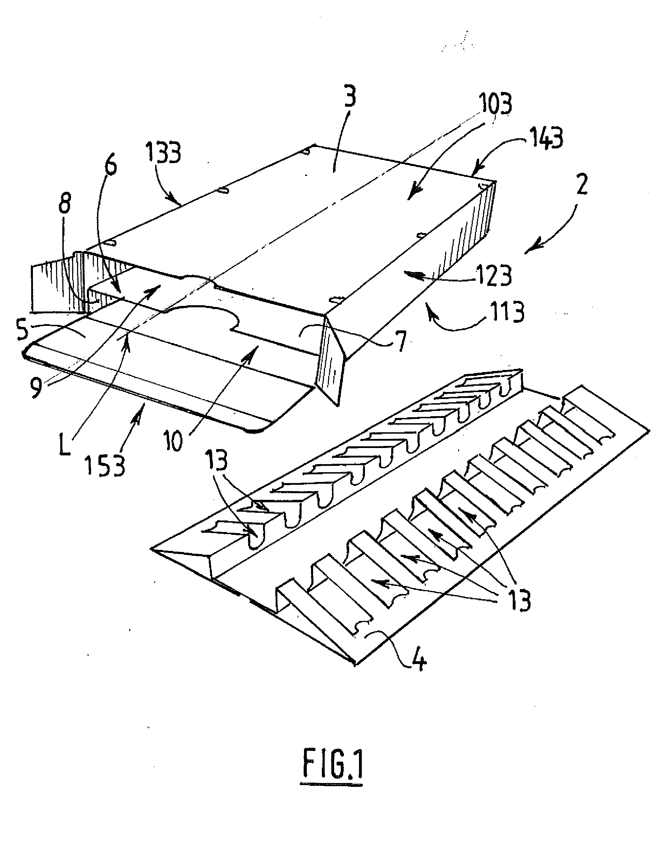

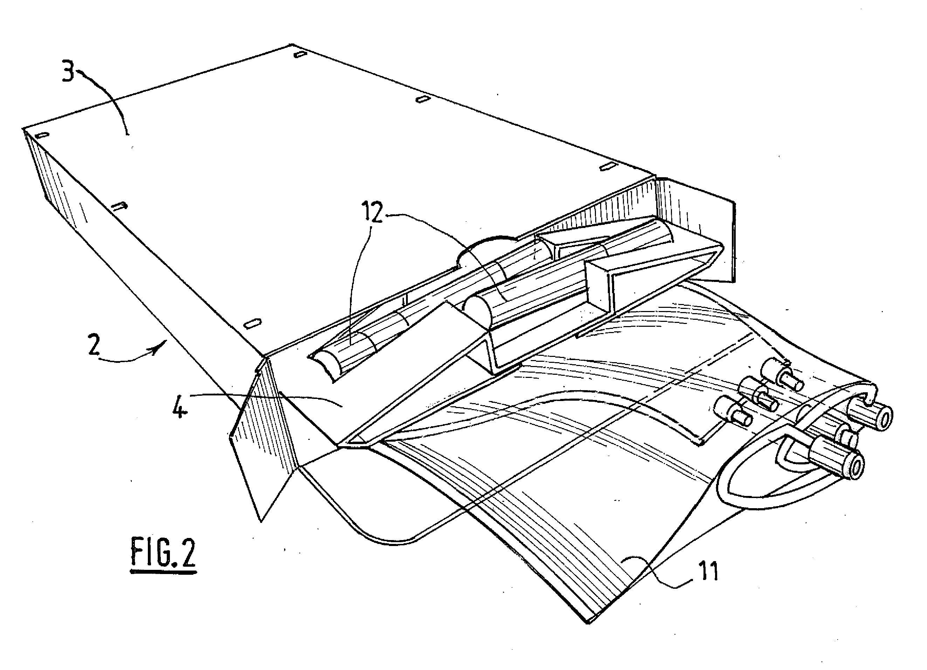

Les figures 1 et 2 représentent un dispositif de conditionnement 2 de produits biologiques réalisé par exemple en carton.

Le dispositif de conditionnement 2 comprend un emballage 3 dans lequel est logé un élément de stockage 4 de tubes de prélèvement de produits biologiques.

L'emballage 3 est constitué par une boîte parallélépipédique. La boîte comprend un couvercle d'ouverture 5 constitué par une face de la boîte présentant un rabat et une zone faisant charnière.

L'emballage 3 comprend une paroi de séparation 6 disposée à l'intérieur de la boîte et s'étendant sur au moins une partie de la longueur de la boîte. La paroi de séparation 6 comprend par exemple une portion centrale 7 s'étendant parallèlement aux faces supérieure et inférieure de la boîte ainsi que deux portions latérales 8 s'étendant sensiblement parallèlement aux faces latérales de la boîte. Les bords longitudinaux libres des portions latérales 8 prennent appui contre les bords longitudinaux inférieurs de la boîte.

La paroi de séparation 6 est conçue pour diviser la boîte en un premier et un second logements 9, 10 par exemple sensiblement identiques.

Comme représenté sur la figure 2, le premier logement 9 est destiné à recevoir l'élément de stockage 4 tandis que le second logement 10 forme une zone de stockage d'une poche de prélèvement 11 de produits biologiques.

L'élément de stockage 4 est réalisé en une pièce et présente une forme sensiblement rectangulaire. Il doit être noté que la longueur et la largeur de l'élément de stockage 4 sont sensiblement identiques à celles de l'emballage 3.

L'élément de stockage 4 comporte des moyens de fixation de tubes de prélèvement 12 de produits biologiques.

Ces moyens de fixation comportent une première et une seconde rangées opposées de logements 13 destinés à recevoir des tubes de prélèvement 12. Les logements 13 de chaque rangée sont identiques et régulièrement espacés le long de l'élément de stockage 4. Les logements 13 s'étendent perpendiculairement à la direction d'insertion de l'élément de stockage 4 à l'intérieur du logement 9 ménagé dans l'emballage 3.

Il doit être noté que les deux rangées de logements 13 sont décalées longitudinalement de sorte qu'un logement 13 appartenant à une rangée ne se trouve pas en regard d'un logements 13 de l'autre rangée.

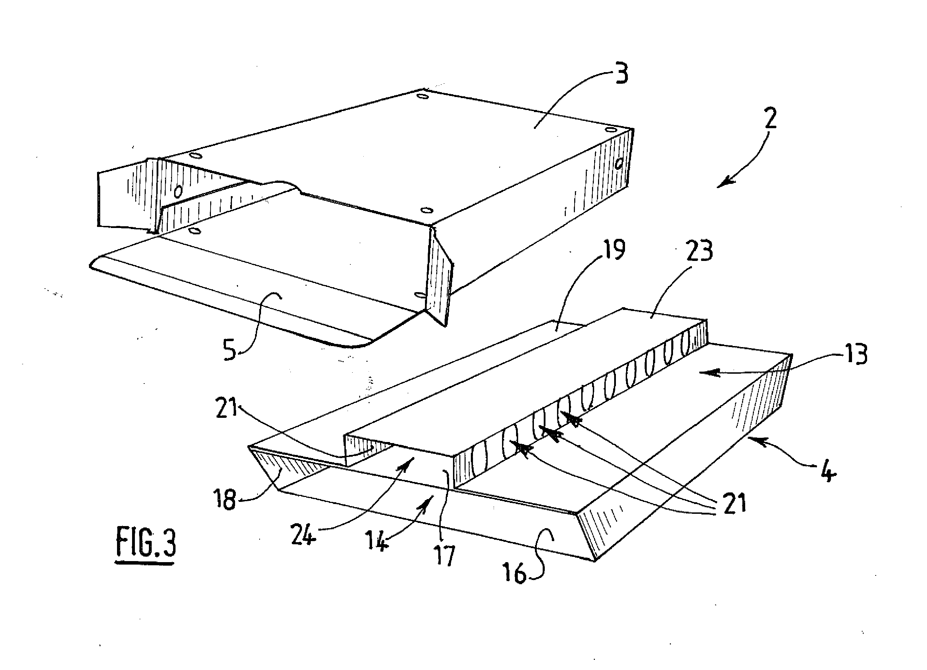

La figure 3 représente une seconde forme d'exécution du dispositif de conditionnement 2 qui diffère de celle représentée sur la figure 1 essentiellement en ce que l'emballage 3 ne comprend pas de paroi de séparation et en ce que l'élément de stockage 4 comporte une première zone de stockage 13 de tubes de prélèvement de produits biologiques et une seconde de zone de stockage 14 d'une poche de prélèvement de produits biologiques.

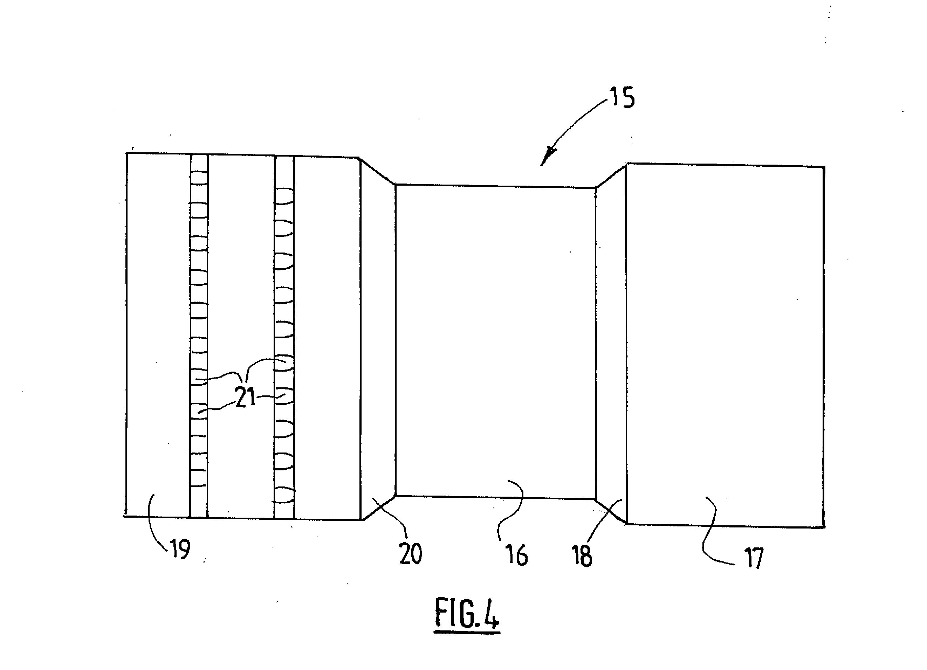

Selon cette forme d'exécution, l'élément de stockage 4 est réalisé en une pièce et obtenu par pliage d'une feuille cartonnée 15 représentée sur la figure 4.

La feuille cartonnée 15 comprend une portion centrale 16 rectangulaire, une première portion latérale rectangulaire 17 reliée à la portion centrale 16 par une première portion trapézoïdale 18, et une seconde portion latérale rectangulaire 19 reliée à la portion centrale 16 par une seconde portion trapézoïdale 20.

Il doit être précisé que les portions 16 et 17 présentent une largeur sensiblement égale à celle de l'emballage 3.

La seconde portion latérale 19 comporte une première et une seconde rangées parallèles d'ouvertures 21 identiques sensiblement circulaires. Les ouvertures 21 de chaque rangée sont régulièrement espacées le long de la seconde portion latérale 19.

Il doit être noté que les ouvertures 21 appartenant à une rangée sont décalées longitudinalement par rapport aux ouvertures 21 de l'autre rangée.

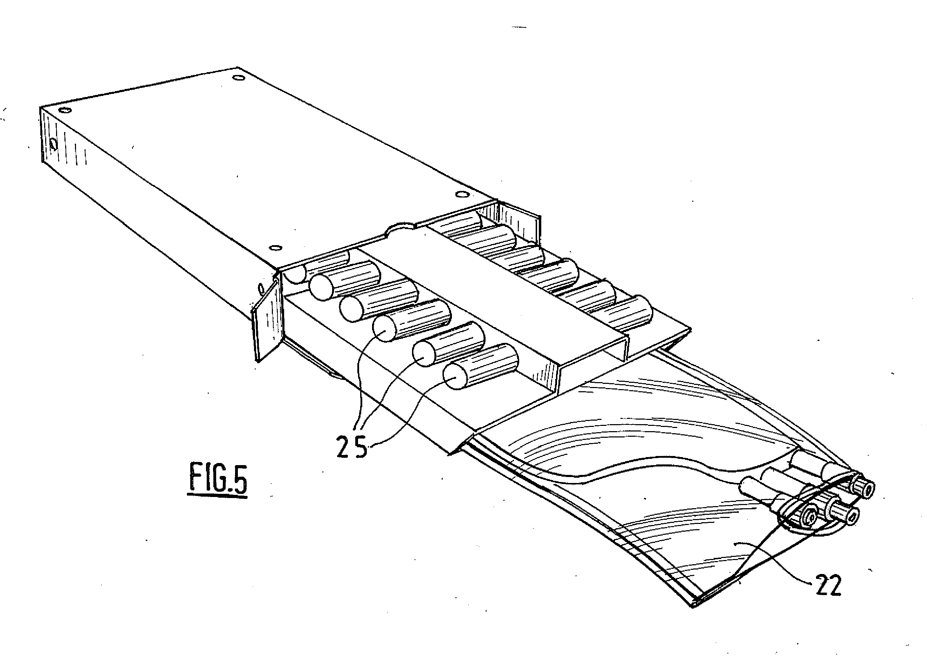

Comme représentée sur les figures 3 et 5, la feuille 15 est conçue pour délimiter, après pliage, d'une part un logement inférieur de section sensiblement rectangulaire constituant la seconde zone de stockage 14 et destiné à recevoir une poche de prélèvement 22 de produits biologiques, et d'autre part un épaulement central 23 disposé au dessus de la seconde zone de stockage 14 et délimitant un logement central 24 de section sensiblement rectangulaire destiné à recevoir des tubes de prélèvement 25 de produits biologiques.

Comme représentée sur la figure 3, la seconde zone de stockage 14 est délimitée par la portion centrale 16, la portion latérale 17 et les deux portions trapézoïdale 18, 20 de la feuille 15 tandis que la première zone de stockage 13 est délimitée par les portions latérales 17 et 19 de la feuille 15.

La portion latérale 19 est pliée de manière à former, avec la portion latérale 17, le logement central 24 disposé au dessus de la seconde zone de stockage 14.

Comme montré sur la figure 5, en conditions d'utilisation, les ouvertures 21 ménagées sur la portion latérale 19 constituent des moyens de fixation de tubes de prélèvement 25 de produits réactifs.

Chaque ouverture 21 permet l'insertion d'une partie d'un tube de prélèvement 25 dans le logement 24 selon une direction perpendiculaire à la direction d'insertion de l'élément de stockage 4 à l'intérieur de l'emballage 3.

Il doit être noté que les deux zones de stockage 13, 14 sont séparées l'une de l'autre par la portion latérale 17 qui forme ainsi une paroi de séparation.

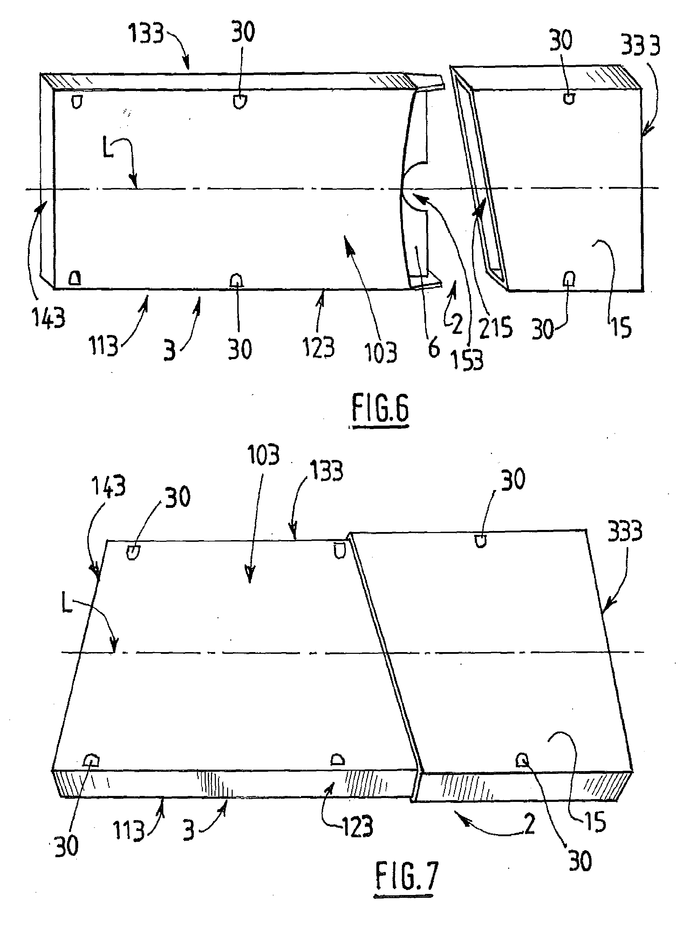

Les figures 6 à10 illustrent une autre forme de réalisation possible de l'invention.

La variante des figures 6 à 10 se distingue de celle de la figure 1 en ce que l'ouverture de l'emballage 3 (boîte parallélépipédique) est refermable via un capuchon 15 distinct apte à être enfilé sur une extrémité de l'emballage 3 (figure 7) ou être séparé (figure 6) pour permettre l'introduction/le retrait de l'élément de stockage 4 et/ou d'une poche de prélèvement 11 par exemple.

Comme représenté, la bordure ouverte 215 du capuchon 15 peut être biseautée par rapport à l'axe perpendiculaire à la direction longitudinale (L) de la boîte pour faciliter son enfilement sur la boîte 3 (à cet effet le capuchon 15 a une forme complémentaire de la boîte).

De même la boîte 3 et/ou le capuchon 15 peuvent comporter des orifices 30 répartis sur la longueur pour permettre l'entrée de liquide cryogénique lorsque l'emballage est stocké dans un réservoir cryogénique et permettre également sa sortie (écoulement) lorsque l'emballage est prélevé par un utilisateur. Ces orifices permettent de maintenir les produits stockés à une température adéquate et homogène.

Des inscriptions peuvent être formées sur tout ou partie des faces 103, 113, 123, 133, 143 de l'emballage 3 et/ou du capuchon 15 (définition et référence des produits stockés dans chaque logement par exemple).

La paroi 6 de séparation de l'emballage peut faire saillie du côté de l'ouverture 153 et peut comporter également une découpe permettant une meilleure préhension manuelle des objets dans chacun des deux logements 9, 10.

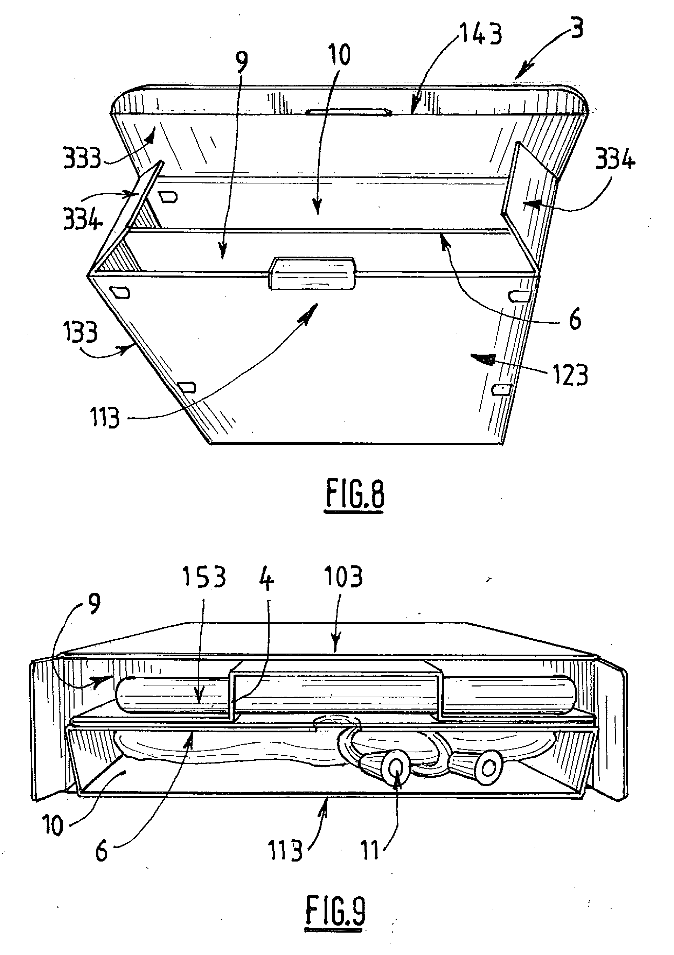

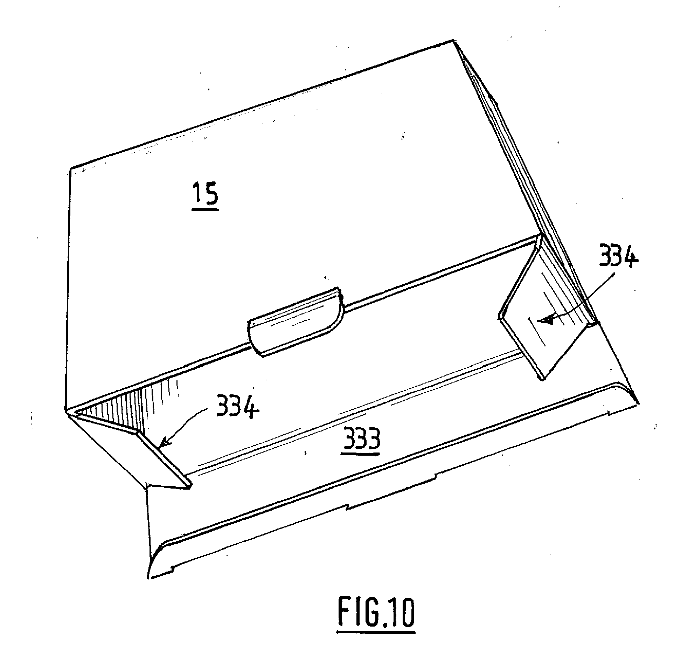

Comme représenté aux figures 8 et 10, l'emballage 3 et/ou le couvercle 15 peuvent être réalisés par une ou plusieurs feuilles repliées.

Par exemple, l'extrémité refermée de l'emballage 153 et/ou du capuchon 15 peut être obtenue par un ou plusieurs rabats 333, 334 solidaire d'une face via une zone faisant charnière.

C'est-à-dire que les faces d'extrémités closes de l'emballage 253, respectivement du couvercle 333, peuvent être sélectivement refermables via des rabats solidaires de l'emballage (respectivement du couvercle 15)

Comme il va de soi, l'invention ne se limite pas aux seules formes d'exécution de ce dispositif de conditionnement, décrites ci-dessus à titre d'exemples, elle en embrasse au contraire toutes les variantes de réalisation.

## Revendications

1. Dispositif de conditionnement (2) de produits biologiques comprenant un emballage (3) parallélépipédique ayant une face supérieure (103), une face inférieure (113), et deux faces latérales (123, 133) s'étendant selon une direction longitudinale (L) entre deux faces d'extrémité (143, 153) et définissant au moins un premier logement (9), le dispositif comportant un élément de stockage (4) distinct destiné à être logé de façon amovible dans le premier logement (9) de l'emballage (3) selon une direction parallèle à la direction longitudinale (L), l'élément de stockage (4) comprenant une zone de stockage d'un premier type de contenant de produits biologiques, l'élément de stockage (4) et/ou l'emballage (3) étant conformés pour délimiter au moins un second logement (10) pour un second type de contenant de produits biologiques, l'emballage (3) comprenant une ouverture sur une face d'extrémité (153) prévue pour l'introduction ou la sortie de l'élément de stockage (4) et/ou du second type de contenant, le dispositif comprenant un couvercle distinct de l'emballage (3) pour obturer sélectivement ladite ouverture.

2. Dispositif de conditionnement (2) selon la revendication 1, **caractérisé en ce que** les deux zones de stockage des premier et second types de contenant sont séparées l'une de l'autre par au moins un élément de séparation (6).

3. Dispositif de conditionnement (2) selon l'une quelconque des revendications 1 et 2, **caractérisé en ce que** l'emballage (3) comprend une paroi de séparation (6) conçue pour diviser l'emballage (3) en au moins un premier et un second logements (9, 10), le premier logement (9) étant destiné à recevoir l'élément de stockage (4) et le second logement (10) formant la zone de stockage du second type de contenant de produits biologiques.

4. Dispositif de conditionnement (2) selon la revendication 3, **caractérisé en ce que** la paroi de séparation (6) s'étendant selon la direction longitudinale (L) sur sensiblement toute la longueur de l'emballage (3).

5. Dispositif de conditionnement (2) selon la revendication 1, **caractérisé en ce que** l'élément de stockage (4) comprend une première zone de stockage (13) du premier type de contenant de produits biologiques et une seconde zone de stockage (14) du second type de contenant de produits biologiques.

6. Dispositif de conditionnement (2) selon la revendication 5, **caractérisé en ce que** la seconde zone de stockage (14) est délimitée par un logement ménagé dans l'élément de stockage (4).

7. Dispositif de conditionnement (2) selon l'une quelconque des revendications 5 et 6, **caractérisé en ce que** l'élément de stockage (4) est réalisé en une pièce et obtenu par pliage d'une feuille (15) comprenant une portion centrale (16), une première portion latérale (17) reliée à la portion centrale (16) par une première portion intermédiaire (18), et une seconde portion latérale (19) reliée à la portion centrale (16) par une seconde portion intermédiaire (20), la seconde zone de stockage (14) étant délimitée, après pliage de la feuille (15), par la portion centrale (16), la première portion latérale (17) et les deux portions intermédiaires (18, 20).

8. Dispositif de conditionnement (2) selon la revendication 7, **caractérisé en ce que** la première zone de stockage (13) est délimitée, après pliage de la feuille (15), par les première et seconde portions latérales (17, 18), et **en ce que** la seconde portion latérale (19) comporte au moins une rangée d'ouvertures (21) destinées à former des moyens de fixation du premier type de contenant.

9. Dispositif de conditionnement (2) selon l'une quelconque des revendications 1 à 8, **caractérisé en ce que** la zone de stockage (10, 14) du second type de contenant de produits biologiques est conçue pour recevoir une poche de prélèvement (11, 22) de produits biologiques.

10. Dispositif de conditionnement (2) selon l'une quelconque des revendications 1 à 9, **caractérisé en ce que** l'élément de stockage (4) comprend des organes de fixation (13, 21) du premier type de contenant de produits biologiques, ces organes de fixation étant agencés pour recevoir au moins un contenant tubulaire, tel qu'un tube de prélèvement (12, 25) de produits biologiques.

11. Dispositif selon l'une quelconque des revendications 1 à 10, **caractérisé en ce que** le couvercle comprend un capuchon (15) ayant la forme d'un tube obturé à une de ses extrémités et conformé pour recouvrir sélectivement l'ouverture et une portion de l'emballage (3) selon la direction longitudinale (L).

12. Dispositif selon l'une quelconque des revendications 1 à 11, **caractérisé en ce que** l'emballage (3) a la forme générale d'un tube clos à une de ses extrémités, les faces d'extrémité de l'emballage ayant chacune une surface inférieure ou égale à la surface de chacune des autres faces de l'emballage (3).

13. Dispositif selon l'une quelconque des revendications 1 à 13, **caractérisé en ce qu'**au moins une partie de la paroi de séparation (6) fait saillie par rapport à l'une des faces de l'emballage selon la direction (L) longitudinale au niveau de l'ouverture.

14. Dispositif selon l'une quelconque des revendications 1 à 17, **caractérisé en ce que** l'emballage (3) et/ou le capuchon (15) comporte des orifices (30) répartis selon la direction longitudinale (L), de préférence à proximité des faces latérales (133, 143), pour permettre la circulation de fluide cryogénique dans le dispositif.

15. Utilisation d'un dispositif selon l'une quelconque des revendications précédentes pour le stockage de deux types distincts de produits biologiques.
